# EUROPEAN PATENT SPECIFICATION

(11) **EP 3 893 853 B1**
(45) Date of publication and mention of the grant of the patent: **28.08.2024**
(21) Application number: 19836523.1
(22) Date of filing: 12.12.2019
(51) Int. Cl.: A61K 9/20, A61K 9/24, A61K 9/28

(54) **SOLID ORAL PHARMACEUTICAL COMPOSITIONS COMPRISING COMPLEX MONOLITHIC MATRICES FOR CHRONOTROPIC ADMINISTRATION OF MEDICAMENTS IN THE GASTROINTESTINAL TRACT**
FESTE ORALE PHARMAZEUTISCHE ZUSAMMENSETZUNGEN MIT KOMPLEXEN MONOLITHISCHEN MATRIZEN FÜR DIE CHRONOTROPE VERABREICHUNG VON MEDIKAMENTEN IM MAGEN-DARM-TRAKT
COMPOSITIONS PHARMACEUTIQUES ORALES SOLIDES COMPRENANT DES MATRICES MONOLITHIQUES COMPLEXES POUR L'ADMINISTRATION CHRONOTROPIQUE DE MÉDICAMENTS DANS LE TRACTUS GASTRO-INTESTINAL

(30) Priority: 14.12.2018 IT 201800011125
(43) Date of publication of application: 20.10.2021
(73) Proprietor: DPL Pharma S.p.A., 20089 Rozzano (MI) (IT)
(72) Inventor: PEDRANI, Massimo, 6815 MELIDE (CH); CONTI, Chiara, 29010 VIGOLO MARCHESE (PC) (IT); GIAMMILLARI, Salvatore Agostino, 20089 ROZZANO (MI) (IT); MACCARI, Giuseppe, 27058 VOGHERA (PV) (IT)
(74) Representative: Bianchetti & Minoja with Trevisan & Cuonzo IPS SRL
(86) International application number: PCT/IB2019/060693
(87) International publication number: WO 2020/121234

(56) References cited:
- WO-A2-2013/098831
- US-A1- 2010 285 125
- CHINTAN PARMAR ET AL: "Formulation and optimization of enteric coated bilayer tablets of mesalamine by RSM: In vitro - In vivo investigations and roentogenographic study", JOURNAL OF DRUG DELIVERY SCIENCE AND TECHNOLOGY, vol. 44, 1 April 2018 (2018-04-01), FR, pages 388 - 398, XP055613467, ISSN: 1773-2247, DOI: 10.1016/j.jddst.2018.01.008

## Description

The present invention relates to solid oral controlled-release pharmaceutical compositions comprising a core consisting of a complex monolithic matrix comprising at least one low/medium viscosity hydroxypropyl methylcellulose, at least one medium/high viscosity hydroxypropyl methylcellulose, one or more methacrylic polymers or copolymers and/or cellulose acetate phthalate and/or hydroxypropyl methylcellulose acetate succinate or shellac, and an outer coating of said core consisting of a layer comprising ethylcellulose, or of a gastroresistant layer or of a layer comprising ethylcellulose coated in turn with gastroresistant polymers.

### Prior art

The treatment of illnesses affected by circadian rhythms, such as "early morning disorders", requires a pre-set concentration of medicament, available at scheduled times. Disorders with a specific circadian cycle exhibit a marked change in symptoms during the day, usually with peaks in the morning and a gradual reduction during the day. The design of compositions able to release a medicament with timing suitable to ensure optimum treatment of disorders involving circadian variations requires full understanding of the absorption, distribution, metabolisation and elimination of the medicaments. Other disorders require site-specific release of medicaments to optimise their efficacy and reduce their adverse effects. Time-specific and site-specific release is achieved by exploiting variations in pH and the different transit times of the medicaments in the gastrointestinal apparatus. It is well known that gastric voiding times can be highly variable, depending on the type and amount of food eaten, and that the fasting pH remains on average between 1.2 and 3.0. Transit times range from a few minutes to a few hours. In the small intestine, the pH tends to approach neutrality, and the transit time is more constant (about 3 ±1 hours), whereas in the colon, pH values can range from 5.5 to pH 7.0-7.5, and transit times vary considerably from individual to individual, from a few hours to 24-48 hours.

Various controlled-release formulations based on monolithic, multi-particulate or multi-unit matrix or reservoir systems have been described. The technologies used comprise gastroresistant retard systems; slow-release systems (simple matrices); solely pH-dependent release systems; solely pH-independent release systems; pulsatile-release systems (an immediate-release portion combined with a slow, gradual controlled-release portion with a simple matrix); extended-release systems (simple extended-release matrices); and reservoir systems involving the use of containment polymers, acting as semipermeable membranes.

The known formulations, described, for example, in WO200610640, WO2003101421, WO2009125981 and WO2011106416, are mainly characterised by single-component systems wherein the release control effect is determined by a single type of excipient. This can lead to low precision of release of the active ingredient in the site and over time, and high variability of release both *in vitro* and *in vivo.* The common retard forms (gastroresistant and/or lag-time) can also exhibit erratic release in the gastrointestinal tract in the distal part of the ileum and/or the initial part of the colon, rapidly releasing the active ingredient without homogeneous distribution thereof in the gastroenteric, ileocolonic and colonic tracts.

WO200400280, WO2010100657 and WO200658059 report examples of matrices containing both a hydroxypropyl methylcellulose and an acrylic polymer. US20100285125 generically indicates the possibility of obtaining a complex matrix containing different types of hydroxypropyl methylcellulose in a mixture with one or more enteric polymers. However, the formulations actually exemplified are characterised by hydroxypropyl methylcellulose acetate succinate and phthalate matrices not mixed with acrylic polymers/copolymers and/or shellac.

WO201398831 describes controlled-release formulations of nisoldipine comprising a low-viscosity hydroxypropyl methylcellulose, a type A methacrylic acid copolymer, and an enteric coating.

Chintan Parmar et al., in J. Drug Delivery Sci. and Technol., Vol. 44, 01.04.2018, 388-398, describe mesalamine-coated tablets consisting of an immediate-release layer and of a controlled-release layer comprising two types of hydroxypropyl methylcellulose with different viscosities.

The known formulations do not provide the ideal solution to the problem of achieving a gradual, constant colonic release of the medicament continuing for several hours so as to guarantee homogeneous distribution, a reproducible release profile, and a very low coefficient of relative standard deviation.

### Description of the invention

It has now been discovered that the activity of medicaments can be efficiently modulated by reducing their frequency of administration and controlling their release in particular sites of the gastrointestinal tract, using complex matrices consisting of a combination of polymers with different characteristics.

In particular, it has been found that combining at least two types of hydroxypropyl methylcellulose having different viscosities with methacrylic polymers or copolymers and/or cellulose resins or esters or shellac makes it possible to prepare formulations that overcome the limitations of the previously known formulations, in particular as regards the variability of the dissolution profiles over time, with lower relative standard deviation (RSD) values than the formulations described in WO201398831 and by Chintan Parmar et al. in J. Drug Delivery Sci. and Technol., Vol 44, 01.04.2018, 388-398.

The solid oral controlled-release pharmaceutical compositions according to the invention comprise one or more active ingredients in a core, and an outer coating of said core, wherein:
a) the core consists of:
   (i) a monolithic matrix containing an active ingredient, a combination of at least one hydroxypropyl methylcellulose having a viscosity ranging between 3 and
   (i) 5000 mPa.s 2% in H₂O at 20°C, and at least one hydroxypropyl methylcellulose having a viscosity ranging between 13500 and 280,000 mPa.s 2% in H₂O at 20°C, with at least one or more methacrylic polymers/copolymers and/or shellac, cellulose acetate phthalate, hydroxypropyl methylcellulose acetate succinate, or
   (ii) a monolithic matrix as defined above adjacent to an immediate-release layer comprising the same active ingredient as contained in the monolithic matrix;
a) the coating consists of a layer comprising ethylcellulose or of a gastroresistant layer or of a layer comprising ethylcellulose which in turn is coated with gastroresistant polymers.

The core consists of a complex monolithic matrix (i) or a bi-layer system consisting of a complex monolithic matrix (i) adjacent to an immediate-release layer comprising the same active ingredient as contained in the monolithic matrix, or another active ingredient.

The coating consists of a layer comprising ethylcellulose or, in another embodiment of the invention, coating b) consists of a layer comprising ethylcellulose coated with gastroresistant polymers.

In yet another embodiment of the invention, the coating consists of a gastroresistant layer.

The acrylic/methacrylic polymers or copolymers of matrix (i) are preferably selected from a mixture of pH-independent methacrylic ester copolymers, pH-independent ammonium alkyl methacrylate copolymers; amino alkyl methacrylate copolymers soluble up to pH 5.0, methacrylic acid copolymers soluble at pH ≥ 5.5, methacrylic acid copolymers soluble at pH 6.0-7.0; and pH-dependent methacrylic acid copolymers soluble at pH ≥ 7.0.

According to one embodiment of the invention, two or more polymers or copolymers are combined, or acrylic polymers or copolymers are combined with shellac, or the latter replaces said acrylic polymers/copolymers.

The gastroresistant coating can be the conventional type, and typically comprises methacrylic acid copolymers soluble at pH ≥ 5.5. Examples of said copolymers are available on the market (Eudragit). Preferably the combination of polymethacrylate L100 with polymethacrylate S100 at the ratio of 1:10 - 10:1 (preferably 1:1); or L 100/55 soluble at pH ≥ 5.5; or shellac; or acetate phthalates/acetate succinates are used.

In the compositions according to the invention, the hydroxypropyl methylcellulose having a viscosity ranging between 3 and 5000 mPa.s 2% in H₂O at 20°C constitutes 1 to 20% of the weight of the core, the hydroxypropyl methylcellulose having a viscosity ranging between 13500 and 280,000 mPa.s 2% in H₂O at 20°C constitutes 1 to 20% of the weight of the core and the methacrylic polymer/copolymer constitutes 0.1 to 20% of the weight of the core (preferably 0.1% to 2%).

Hydroxypropyl methylcellulose having a viscosity ranging between 3.0 and 5000 mPa.s 2% in H₂O at 20°C is available on the market under the names of Methocel K3LV, K100 LV, E5 premium and K4M.

Hydroxypropyl methylcellulose having a viscosity ranging between 13500 and 280,000 mPa.s 2% in H₂O at 20°C is available on the market under the names of Methocel K15 M, K100 M and K200 M. Methocel K 15 M and K100 M are preferred.

Ethylcellulose is present in the core-coating layer in percentages ranging from 1% to 20% of the weight of the core; preferably 5%.

The matrix core can comprise conventional excipients such as diluents (microcrystalline cellulose, starches, sugars, phosphate salts - hydrated and anhydrous mono/dibasic sodium phosphate), binders (PVP, starches, cellulose, dextrins, maltodextrins, low-viscosity cellulose), glidants (colloidal silicon dioxides), flow agents (talc), lubricants (Mg stearate, fumaryl stearate, stearic acid, glyceryl behenate), disintegrating agents (croscarmellose, sodium starch glycolate, crosslinked polyvinylpyrrolidone, starches) and other functional excipients (waxes, polycarbophil, carbomer, glycerides).

The matrix is prepared by processes of partition and direct compression, dry granulation, compacting, wet granulation, melting and extrusion.

Powders, granules, microgranules, pellets, mini-tablets, tablets, capsules, sachets and sticks can thus be obtained.

The resulting matrix/mini-matrix can then be coated with a gastroresistant film containing pH-dependent polymers that prevent release for at least 2 hours under pH conditions < 1.2-5.5. pH-dependent methacrylic acid copolymers soluble at pH ≥ 5.5 (L 100-55/L 30 D-55); pH-dependent methacrylic acid copolymers soluble at pH 6.0-7.0 (L 100/L 12.5); pH-dependent methacrylic acid copolymers soluble at pH ≥ 7.0 (S 100/S 12.5/FS 30D); shellac; cellulose acetate phthalate; hydroxypropyl methylcellulose acetate succinate may be used.

At a third stage, a core coating can be applied which is alternative and/or additional to and beneath the gastroresistant coating with pH-independent polymers (ethylcellulose or hydroxypropyl methylcellulose with different viscosities), which acts as membranes delaying the passage of the ingredient loaded into the matrix/mini-matrix core following contact with biological fluids.

The matrix is coated with a quantity of polymer sufficient to guarantee that it remains intact in gastric and enteric juices for at least 2-4 hours before the release of the active ingredient from the core (lag time). To reduce the impact of variable gastric voiding times, a further (pH-dependent) gastroresistant coating can be applied outside the (pH-independent) matrix core and outside the (pH-independent) cellulose film coating, to further delay contact between the biological fluids and the modified-release core (extended release).

In this way the system prevents early release during the stomach-jejunum transit time, initiating the modulated-release programme lasting up to 24 hours and ensuring homogeneous distribution of the active ingredient in the duodenum, ileum and distal ileum and in the ascending, transverse and descending tracts of the large intestine.

The use of hydrophilic polymers with different rheological characteristics (viscosity/swelling properties) combined with pH-dependent and/or pH-independent polymers allows the release to be modulated for between 8 and 24 hours. If desired, a modified-, controlled-release core can be combined with an immediate-release layer (bi-layer and/or tri-layer matrix/mini-matrix); a system thus designed gives results of "therapeutic equivalence" or different levels of therapeutic efficacy.

Examples of active ingredients which can be advantageously formulated according to the invention comprise non-steroidal anti-inflammatory drugs, beta blockers, vasodilators, calcium antagonists, angiotensin II receptor antagonists, ACE inhibitors, statins, antidiabetics, hypoglycaemics, incretin mimetics, tranquillisers, antihistamines, antidepressants, antipsychotics, analgesics, antitumorals, antibacterials, antibiotics (such as rifaximin, rifampicin, rifamycin sv, oral beta-lactams, macrolides, quinolone, etc.), antifungals, antihyperlipidaemics, antifibrinolytics, steroidal anti-inflammatory drugs, monoclonal antibodies, antivirals, anticoagulants, antirheumatics, immunosuppressants, immunomodulators, bronchodilators, multiple sclerosis drugs, antihelminthics/antiinflammatories. The formulations according to the invention are particularly suitable to optimise the pharmacological effect of medicaments which have an unfavourable profile in terms of compliance, because of the large number of daily administrations, and the side effects.

The invention is described in detail in the examples below.

### EXAMPLE 1

100 g of triazolam is loaded into a granulator with 7.5 Kg of lactose monohydrate and 3 Kg of microcrystalline cellulose. 1.1 Kg of hydroxypropyl methylcellulose (HPMC K4M, 1.1 Kg of hydroxypropyl methylcellulose (HPMC K100M) and 20 g of polymethacrylate L100-55 are added in sequence to said mixture. The ingredients are mixed until a homogeneous dispersion of active ingredient in the matrices is obtained; 100 g of magnesium stearate and 100 g of talc are then added in sequence. The mixture is then homogenised for at least 15 minutes. This mixture will form part of the first, controlled-release layer of the tablet.

100 g of triazolam is loaded into a second granulator, and 2.5 Kg of lactose monohydrate, 1 Kg of microcrystalline cellulose, 1.16 Kg of crospovidone, 1.16 of croscarmellose, 100 g of magnesium stearate and 100 g of talc are added. The mixture is then homogenised for at least 15 minutes. This mixture will form part of the second, immediate-release layer of the tablet. The two separate mixtures are then compressed to obtain a double-layer tablet weighing 191.4 mg. The resulting tablets are then film-coated with a gastroresistant solution/suspension based on 3.2 Kg of polymethacrylate L100-55, 800 g of talc, 230 g of titanium dioxide and 130 g triethyl citrate, to obtain a tablet with a mean weight of 235 mg.

When subjected to disintegration and dissolution tests at pH 1, the tablets remain intact for at least 2 hours, with release below 1%; when subjected to the dissolution test at pH ≥ 6.4 they exhibit the following release profile: not more than 60% after 60 minutes, at pH 7.2 not more than 70% after 240 minutes, and not more than 80% after 480 minutes; the value must be > 80% after 24 hours.

### EXAMPLE 2

200 g of alprazolam is loaded into a granulator with 10 Kg of lactose monohydrate and 4 Kg of microcrystalline cellulose. 1.1 Kg of hydroxypropyl methylcellulose (HPMC K4M), 1.1 Kg of hydroxypropyl methylcellulose (HPMC K100M), 25 g of polymethacrylate L100 and 25 g of polymethacrylate S100 are added in sequence to the same system. The ingredients are mixed until a homogeneous dispersion of active ingredient in the matrices is obtained; 150 g of magnesium stearate and 200 g of talc are then added in sequence. The mixture is then homogenised for at least 15 minutes. The mixture is then compressed to obtain a mini-tablet weighing 168 mg. The resulting tablets are then film-coated with a gastroresistant solution/suspension based on 600 g of shellac (25% solution), 650 g of talc, 300 g of titanium dioxide and 150 g of triethyl citrate, to obtain a tablet with a mean weight of 185 mg.

When subjected to disintegration and dissolution tests at pH 1, the tablets remain intact for at least 2 hours, with release below 1%; when subjected to the dissolution test at pH ≥ 6.4, they exhibit release below 10%; when subjected to the dissolution test at pH ≥ 7.2, they exhibit the following release profile: not more than 20% after 60 minutes, not more than 60% after 240 minutes, and not more than 80% after 480 minutes; the value must be ≥ 90% after 24 hours.

### EXAMPLE 3

250 g of ramipril is loaded into a granulator with 2.25 Kg of lactose monohydrate and 7.425 Kg of microcrystalline cellulose. 1.5 Kg of hydroxypropyl methylcellulose (HPMC K4M), 1 Kg of hydroxypropyl methylcellulose (HPMC K100M), 50 g of polymethacrylate L100, 50 g of polymethacrylate S100 and 100 g of shellac are added in sequence to the same system. The ingredients are mixed until a homogeneous dispersion of active ingredient in the matrices is obtained, and 150 g of magnesium stearate and 250 g of talc are then added in sequence. The mixture is then homogenised for at least 20 minutes. This mixture will form part of the first, controlled-release layer of the tablet.

250 g of ramipril is loaded into a second granulator, and 750 g of lactose monohydrate, 2.4251 Kg of microcrystalline cellulose, 1.5 Kg of crospovidone, 1.5 of croscarmellose, 150 g of magnesium stearate and 250 g of talc are added. The mixture is then homogenised for at least 20 minutes. This mixture will form part of the second, immediate-release layer of the tablet. The two separate mixtures are then compressed to obtain a double-layer tablet weighing 136.5 mg. The resulting tablets are film-coated with a gastroresistant solution/suspension based on 750 g of polymethacrylate L100, 750 g of polymethacrylate S100, 500 g of talc, 200 g of titanium dioxide and 200 g of triethyl citrate, to obtain a tablet with a mean weight of 222.5 mg.

When subjected to disintegration and dissolution tests at pH 1.2, the tablets remain intact for at least 2 hours, with release below 1%; when subjected to the dissolution test at pH ≥ 6.4, they exhibit release below 1%; when subjected to the dissolution test at pH ≥ 7.2 they exhibit the following release profile: not more than 60% after 60 minutes, not more than 75% after 240 minutes, and not more than 85% after 480 minutes; the value must be > 90% after 24 hours.

### EXAMPLE 4

250 g of ramipril is loaded into a granulator with 2.25 Kg of lactose monohydrate and 7.425 Kg of microcrystalline cellulose. 1.5 Kg of hydroxypropyl methylcellulose (HPMC K4M), 1 Kg of hydroxypropyl methylcellulose (HPMC K100M) and 200 g of polymethacrylate L100-55 are added in sequence to the same system. The ingredients are mixed until a homogeneous dispersion of active ingredient in the matrices is obtained, and 150 g of magnesium stearate and 250 g of talc are then added in sequence. The mixture is then homogenised for at least 20 minutes. This mixture will form part of the first, controlled-release layer of the tablet.

250 g of ramipril is loaded into a second granulator, and 750 g of lactose monohydrate, 2.4251 Kg of microcrystalline cellulose, 1.5 Kg of crospovidone, 1.5 of croscarmellose, 150 g of magnesium stearate and 250 g of talc are added. The mixture is then homogenised for at least 20 minutes. This mixture will form part of the second, immediate-release layer of the tablet. The two separate mixtures are then compressed to obtain a double-layer tablet weighing 136.5 mg. The resulting tablets are then film-coated with a gastroresistant solution/suspension based on 1.5 Kg of polymethacrylate L100-55, 500 g of talc, 200 g of titanium dioxide and 200 g of triethyl citrate, to obtain a tablet with a mean weight of 222.5 mg.

When subjected to disintegration and dissolution tests at pH 1, the tablets remain intact for at least 2 hours, with release ≤ 1%; when subjected to the dissolution test at pH ≥ 6.4, the tablets exhibit a release not exceeding 60% after 60 minutes; when subjected to the dissolution test at pH ≥ 7.2 they exhibit the following release profile: not more than 70% after 60 minutes; not more than 80% after 240 minutes, not more than 90% after 480 minutes; the value must be > 90% after 24 hours.

### EXAMPLE 5

4 Kg of gliclazide is loaded into a granulator with 12 Kg of lactose monohydrate and 2.25 Kg of microcrystalline cellulose. 2.5 Kg of hydroxypropyl methylcellulose (HPMC K4M), 2.5 Kg of hydroxypropyl methylcellulose (HPMC K100M), 50 g of polymethacrylate L100-55 and 50 g of polymethacrylate RL100 are added in sequence to the same system. The ingredients are mixed until a homogeneous dispersion of active ingredient in the matrices is obtained, and 150 g of magnesium stearate and 250 g of talc are then added in sequence. The mixture is then homogenised for at least 15 minutes. This mixture will form part of the first, controlled-release layer of the tablet.

4 Kg of gliclazide is loaded into a second granulator, and 4 Kg of lactose monohydrate, 750 g of microcrystalline cellulose, 1.25 Kg of crospovidone, 1.25 g of croscarmellose, 150 g of magnesium stearate and 250 g of talc are added. The mixture is then homogenised for at least 20 minutes. This mixture will form part of the second, immediate-release layer of the tablet. The two separate mixtures are then compressed to obtain a double-layer tablet weighing 354 mg. The resulting tablets are then film-coated with a gastroresistant solution/suspension based on 1.5 Kg of polymethacrylate L100-55, 500 g of talc, 200 g of titanium dioxide and 200 g of triethyl citrate, to obtain a tablet with a mean weight of 378 mg.

When subjected to disintegration and dissolution tests at pH 1, the tablets remain intact for at least 2 hours, with release ≤ 1%; when subjected to the dissolution test at pH ≥ 6.4, the tablets exhibit a release not exceeding 60% after 60 minutes; when subjected to the dissolution test at pH ≥ 7.2 they exhibit the following release profile: not more than 70% after 60 minutes; not more than 80% after 240 minutes, not more than 90% after 480 minutes; the value must be > 90% after 24 hours.

### EXAMPLE 6

3.125 Kg of metformin is loaded into a granulator with 3.25 Kg of microcrystalline cellulose. 250 g of hydroxypropyl methylcellulose (HPMC K4M), 125 g of hydroxypropyl methylcellulose (HPMC K15M), 10 g of polymethacrylate RL100 and 10 g of polymethacrylate RS100 are added in sequence to the resulting mixture. The ingredients are mixed until a homogeneous dispersion of active ingredient in the matrices is obtained, and the mixture is then granulated with an aqueous solution containing 150 g of polyvinylpyrrolidone. After drying, 30 g of magnesium stearate and 15 g of talc are added in sequence. The mixture is then homogenised for at least 15 minutes. This mixture will form part of the first, controlled-release layer of the mini-tablet.

3.125 Kg of metformin is loaded into a second granulator and granulated with an aqueous solution containing 150 g of polyvinylpyrrolidone. After drying, 335 g of crospovidone, 335 g of croscarmellose, 30 g of magnesium stearate, 75 g of talc and 10 g of colloidal silicon dioxide are added and mixed homogeneously. The mixture is then homogenised for at least 20 minutes. This mixture will form part of the second, immediate-release layer of the mini-tablet. The two separate mixtures are then compressed to obtain a 5 mm double-layer mini-tablet weighing 81 mg. The resulting mini-tablets are then film-coated with a gastroresistant solution/suspension based on 540 g of polymethacrylate L100-55, 200 g of talc, 10 g of titanium dioxide and 60 g of triethyl citrate, to obtain a mini-tablet with a mean weight of 90 mg.

When subjected to disintegration and dissolution tests at pH 1, the tablets remain intact for at least 2 hours, with release ≤ 1%; when subjected to the dissolution test at pH ≥ 6.4, the tablets exhibit a release not exceeding 50% after 60 minutes; when subjected to the dissolution test at pH ≥ 7.2 they exhibit the following release profile: not more than 70% after 60 minutes; not more than 80% after 240 minutes, not more than 90% after 480 minutes; the value must be > 90% after 24 hours.

### EXAMPLE 7

500 g of atenolol is loaded into a granulator with 500 g of microcrystalline cellulose and 1.225 g of dicalcium phosphate. 225 g of hydroxypropyl methylcellulose (HPMC K4M), 225 g of hydroxypropyl methylcellulose (HPMC K15M) and 4.5 g of polymethacrylate L100-55 are added in sequence to said mixture. The ingredients are mixed until a homogeneous dispersion of active ingredient in the matrices is obtained. 13 g of magnesium stearate and 22.5 g of talc are then added in sequence. The mixture is then homogenised for at least 15 minutes. This mixture will form part of the first, controlled-release layer of the mini-tablet.

500 g of atenolol is loaded into a second granulator. 500 g of microcrystalline cellulose, 225 g of lactose monohydrate, 225 g of crospovidone, 225 g of croscarmellose, 13 g of magnesium stearate and 27 g of talc are added and homogeneously mixed. The mixture is then homogenised for at least 20 minutes. This mixture will form part of the second, immediate-release layer of the mini-tablet. The two separate mixtures are then compressed to obtain a 4 mm double-layer mini-tablet weighing 44.3 mg. The resulting mini-tablets are then film-coated with a solution of 40 g of HPMC 5 premium, 20 g of talc, 29 g of titanium dioxide and 14.9 g of triethyl citrate, to obtain a mini-tablet with a mean weight of 46 mg.

When subjected to the dissolution test at pH 1 and the dissolution test at pH ≥ 6.0, the tablets exhibited the following release profile: not more than 60% after 60 minutes, not more than 75% after 240 minutes, and not more than 85% after 480 minutes; the value must be > 90% after 24 hours.

### EXAMPLE 8

500 g of atenolol is loaded into a granulator with 500 g of microcrystalline cellulose and 1.225 of dicalcium phosphate. 225 g of hydroxypropyl methylcellulose (HPMC K4M), 225 g of hydroxypropyl methylcellulose (HPMC K15M) and 4.5 g of polymethacrylate L100-55 are added in sequence to said mixture. The ingredients are mixed until a homogeneous dispersion of active ingredient in the matrices is obtained. 13 g of magnesium stearate and 22.5 g of talc are then added in sequence. The mixture is then homogenised for at least 15 minutes. This mixture will form part of the first, controlled-release layer of the mini-tablet.

500 g of atenolol is loaded into a second granulator. 500 g of microcrystalline cellulose, 225 g of lactose monohydrate, 225 g of crospovidone, 225 g of croscarmellose, 13 g of magnesium stearate and 27 g of talc are added and homogeneously mixed. The mixture is then homogenised for at least 20 minutes. This mixture will form part of the second, immediate-release layer of the mini-tablet. The two separate mixtures are then compressed to obtain a 4 mm double-layer mini-tablet weighing 44.3 mg. The resulting mini-tablets are then film-coated with a gastroresistant solution of 169 g of polymethacrylate L100-55, 20 g of talc, 29 g of titanium dioxide and 14.9 g of triethyl citrate, to obtain a mini-tablet with a mean weight of 47.3 mg.

When subjected to disintegration and dissolution tests at pH 1, the tablets remain intact for at least 2 hours, with release ≤ 1%; when subjected to the dissolution test at pH ≥ 6.4, the tablets exhibit a release not exceeding 50% after 60 minutes; when subjected to the dissolution test at pH ≥ 7.2 they exhibit the following release profile: not more than 65% after 60 minutes; not more than 80% after 240 minutes, not more than 90% after 480 minutes; the value must be > 90% after 24 hours.

### EXAMPLE 9

100 g of triazolam (F9) is loaded into a granulator with 7.5 Kg of lactose monohydrate and 3 Kg of microcrystalline cellulose. 1.1 Kg of hydroxypropyl methylcellulose (HPMC K4M), 1.1 Kg of hydroxypropyl methylcellulose (HPMC K100M) and 20 g of polymethacrylate L100-55 are added in sequence to said mixture. The ingredients are mixed until a homogeneous dispersion of active ingredient in the matrices is obtained; 100 g of magnesium stearate and 100 g of talc are then added in sequence. The mixture is then homogenised for at least 15 minutes. This mixture will form part of the first, controlled-release layer of the tablet.

100 g of triazolam is loaded into a second granulator, and 2.5 Kg of lactose monohydrate, 1 Kg of microcrystalline cellulose, 1.16 Kg of crospovidone, 1.16 Kg of croscarmellose, 100 g of magnesium stearate and 100 g of talc are added. The mixture is then homogenised for at least 15 minutes. This mixture will form part of the second, immediate-release layer of the tablet. The two separate mixtures are then compressed to obtain a double-layer tablet weighing 191.4 mg. The resulting tablets are then film-coated with a solution/suspension containing 1.24 Kg of ethylcellulose, 800 g of talc, 230 g of titanium dioxide and 130 g of triethyl citrate, to obtain a tablet with a mean weight of 215.4 mg.

When subjected to disintegration and dissolution tests at pH 1, the tablets remain intact for at least 2 hours, with release below 1%; at pH ≥ 6.4 they exhibit release ≤ 10% after 60 minutes; at pH 7.2 release ≤ 50% after 60 minutes; release ≤ 60% after 240 minutes, and not more than 80% after 480 minutes; the value must be > 90% after 18 hours.

### EXAMPLE 10

200 g of alprazolam (F10) is loaded into a granulator with 10 Kg of lactose monohydrate and 4 Kg of microcrystalline cellulose. 1.1 Kg of hydroxypropyl methylcellulose (HPMC K4M), 1.1 Kg of hydroxypropyl methylcellulose (HPMC K100M), 25 g of polymethacrylate L100 and 25 g of polymethacrylate S100 are added in sequence to the same system. The ingredients are mixed until a homogeneous dispersion of active ingredient in the matrices is obtained; 150 g of magnesium stearate and 200 g of talc are then added in sequence. The mixture is then homogenised for at least 15 minutes. The mixture is then compressed to obtain a mini-tablet weighing 168 mg. The resulting tablets are then film-coated with a solution/suspension containing 700 g of ethylcellulose, 650 g of talc, 300 g of titanium dioxide and 150 g of triethyl citrate, to obtain a tablet with a mean weight of 186 mg.

When subjected to disintegration and dissolution tests at pH 1, the tablets remain intact for at least 2 hours, with release below 1%; at pH ≥ 6.4 they exhibit release below 10%; when subjected to the dissolution test at pH ≥ 7.2 they exhibit the following release profile: not more than 20% after 60 minutes, not more than 60% after 240 minutes, and not more than 80% after 480 minutes; the value must be > 90% after 18 hours.

### EXAMPLE 11

250 g of ramipril is loaded into a granulator with 2.25 g of lactose monohydrate and 7.425 Kg of microcrystalline cellulose. 1.5 Kg of hydroxypropyl methylcellulose (HPMC K4M), 1 Kg of hydroxypropyl methylcellulose (HPMC K100M), 50 g of polymethacrylate L100, 50 g of polymethacrylate S100 and 100 g of shellac are added in sequence to the same system. The ingredients are mixed until a homogeneous dispersion of active ingredient in the matrices is obtained, and 150 g of magnesium stearate and 250 g of talc are then added in sequence. The mixture is then homogenised for at least 20 minutes. This mixture will form part of the first, controlled-release layer of the tablet.

250 g of ramipril is loaded into a second granulator, and 750 Kg of lactose monohydrate, 2.4251 Kg of microcrystalline cellulose, 1.5 Kg of crospovidone, 1.5 Kg of croscarmellose, 150 g of magnesium stearate and 250 g of talc are added. The mixture is then homogenised for at least 20 minutes. This mixture will form part of the second, immediate-release layer of the tablet. The two separate mixtures are then compressed to obtain a double-layer tablet weighing 136.5 mg. The resulting tablets are then film-coated with a solution/suspension containing 700 g of ethylcellulose, 500 g of talc, 200 g of titanium dioxide and 200 g of triethyl citrate, to obtain a tablet with a mean weight of 214.5 mg.

When subjected to disintegration and dissolution tests at pH 1, the tablets remain intact for at least 2 hours, with release below 1%; at pH ≥ 6.4 they exhibit release ≤ 10% after 60 minutes; at pH 7.2 release ≤ 60% after 60 minutes; release ≤ 60% after 240 minutes, and not more than 80% after 480 minutes; the value must be > 90% after 18 hours.

### EXAMPLE 12

250 g of ramipril (F12) is loaded into a granulator with 2.25 Kg of lactose monohydrate and 7.425 Kg of microcrystalline cellulose. 1.5 Kg of hydroxypropyl methylcellulose (HPMC K4M), 1 Kg of hydroxypropyl methylcellulose (HPMC K100M) and 200 g of polymethacrylate L100-55 are added in sequence to the same system. The ingredients are mixed until a homogeneous dispersion of active ingredient in the matrices is obtained, and 150 g of magnesium stearate and 250 g of talc are then added in sequence. The mixture is then homogenised for at least 20 minutes. This mixture will form part of the first, controlled-release layer of the tablet.

250 g of ramipril is loaded into a second granulator, and 750 g of lactose monohydrate, 2.4251 Kg of microcrystalline cellulose, 1.5 Kg of crospovidone, 1.5 Kg of croscarmellose, 150 g of magnesium stearate and 250 g of talc are added. The mixture is then homogenised for at least 20 minutes. This mixture will form part of the second, immediate-release layer of the tablet. The two separate mixtures are then compressed to obtain a double-layer tablet weighing 136.5 mg. The resulting tablets are then film-coated with a solution/suspension containing 1 Kg of ethylcellulose, 300 g of talc, 200 g of titanium dioxide and 200 g of triethyl citrate, to obtain a tablet with a mean weight of 215.5 mg.

When subjected to disintegration and dissolution tests at pH 1, the tablets remain intact for at least 2 hours, with release below 1%; at pH ≥ 6.4 they exhibit release ≤ 5% after 60 minutes; at pH 7.2 release ≤ 50% after 60 minutes; release ≤ 60% after 240 minutes, and not more than 80% after 480 minutes; after 18 hours the value is > 90%.

### EXAMPLE 13

4 Kg of gliclazide is loaded into a granulator with 12 Kg of lactose monohydrate and 2.25 Kg of microcrystalline cellulose. 2.5 Kg of hydroxypropyl methylcellulose (HPMC K4M), 2.5 Kg of hydroxypropyl methylcellulose (HPMC K100M), 50 g of polymethacrylate L100-55 and 50 g of polymethacrylate RL100 are added in sequence to the same system. The ingredients are mixed until a homogeneous dispersion of active ingredient in the matrices is obtained, and 150 g of magnesium stearate and 250 g of talc are then added in sequence. The mixture is then homogenised for at least 15 minutes. This mixture will form part of the first, controlled-release layer of the tablet.

4 Kg of gliclazide is loaded into a second granulator, and 4 Kg of lactose monohydrate, 750 g of microcrystalline cellulose, 1.25 Kg of crospovidone, 1.25 Kg of croscarmellose, 150 g of magnesium stearate and 250 g of talc are added. The mixture is then homogenised for at least 20 minutes. This mixture will form part of the second, immediate-release layer of the tablet. The two separate mixtures are then compressed to obtain a double-layer tablet weighing 354 mg. The resulting tablets are then film-coated with a solution/suspension containing 1.2 Kg of ethylcellulose, 500 g of talc, 200 g of titanium dioxide and 200 g of triethyl citrate, to obtain a tablet with a mean weight of 375 mg.

When subjected to disintegration and dissolution tests at pH 1, the tablets remain intact for at least 2 hours, with release below 1%; at pH ≥ 6.4 they exhibit release ≤ 5% after 60 minutes; at pH 7.2 release ≤ 45% after 60 minutes; release ≤ 60% after 240 minutes, and not more than 85% after 480 minutes; the value must be > 90% after 18 hours.

### EXAMPLE 14

3.125 Kg of metformin is loaded into a granulator with 3.25 Kg of microcrystalline cellulose. 250 g of hydroxypropyl methylcellulose (HPMC K4M), 125 g of hydroxypropyl methylcellulose (HPMC K15M), 10 g of polymethacrylate RL100 and 10 g of polymethacrylate RS100 are added in sequence to the resulting mixture. The ingredients are mixed until a homogeneous dispersion of active ingredient in the matrices is obtained, and the mixture is then granulated with an aqueous solution containing 150 g of polyvinylpyrrolidone. After drying, 30 g of magnesium stearate, 15 g of talc and 10 g of colloidal silicon dioxide are added in sequence. The mixture is then homogenised for at least 15 minutes. This mixture will form part of the first, controlled-release layer of the mini-tablet.

3.125 Kg of metformin is loaded into a second granulator, and granulated with an aqueous solution containing 150 g of polyvinylpyrrolidone. After drying, 335 g of crospovidone, 335 g of croscarmellose, 30 g of magnesium stearate and 75 g of talc are added and mixed homogeneously. The mixture is then homogenised for at least 20 minutes. This mixture will form part of the second, immediate-release layer of the mini-tablet. The two separate mixtures are then compressed to obtain a 5 mm double-layer mini-tablet weighing 78.8 mg. The resulting mini-tablets are then film-coated with a solution/suspension containing 270 g of ethylcellulose, 85.5 g of talc, 29 g of titanium dioxide and 15.5 g of triethyl citrate, to obtain a mini-tablet with a mean weight of 88.7 mg.

When subjected to disintegration and dissolution tests at pH 1, the mini-tablets remain intact for at least 2 hours, with release below 1%; at pH ≥ 6.4 they exhibit release ≤ 10% after 60 minutes; at pH 7.2 release ≤ 60% after 60 minutes; release ≤ 70% after 240 minutes, and not more than 85% after 480 minutes; the value must be > 90% after 18 hours.

### EXAMPLE 15

500 g of atenolol is loaded into a granulator with 500 g of microcrystalline cellulose and 1.225 g of dicalcium phosphate. 225 g of hydroxypropyl methylcellulose (HPMC K4M, 225 g of hydroxypropyl methylcellulose (HPMC K15M) and 4.5 g of polymethacrylate L100-55 are added in sequence to said mixture. The ingredients are mixed until a homogeneous dispersion of active ingredient in the matrices is obtained. 13 g of magnesium stearate and 22.5 g of talc are then added in sequence. The mixture is then homogenised for at least 15 minutes. This mixture will form part of the first, controlled-release layer of the mini-tablet.

500 g of atenolol is loaded into a second granulator. 500 g of microcrystalline cellulose, 225 g of lactose monohydrate, 225 g of crospovidone, 225 g of croscarmellose, 13 g of mg stearate and 27 g of talc are added and homogeneously mixed. The mixture is then homogenised for at least 15 minutes. This mixture will form part of the second, immediate-release layer of the mini-tablet. The two separate mixtures are then compressed to obtain a 4 mm double-layer mini-tablet weighing 45.4 mg. The resulting mini-tablets are then film-coated with a solution/suspension containing 270 g of ethylcellulose, 85.5 g of talc, 29 g of titanium dioxide and 15.5 g of triethyl citrate, to obtain a mini-tablet with a mean weight of 48.4 mg.

When subjected to disintegration and dissolution tests at pH 1, the mini-tablets remain intact for at least 2 hours, with release below 1%; at pH ≥ 6.4 they exhibit release ≤ 15% after 60 minutes; at pH 7.2 release ≤ 60% after 60 minutes; release ≤ 70% after 240 minutes, and not more than 85% after 480 minutes; the value must be > 90% after 18 hours.

### EXAMPLE 16

500 g of atenolol is loaded into a granulator with 500 g of microcrystalline cellulose and 1.225 g of dicalcium phosphate. 225 g of hydroxypropyl methylcellulose (HPMC K4M), 225 g of hydroxypropyl methylcellulose (HPMC K15M) and 4.5 g of polymethacrylate L100-55 are added in sequence to said mixture. The ingredients are mixed until a homogeneous dispersion of active ingredient in the matrices is obtained. 13 g of magnesium stearate and 22.5 g of talc are then added in sequence. The mixture is then homogenised for at least 15 minutes. This mixture will form part of the first, controlled-release layer of the mini-tablet.

500 g of atenolol is loaded into a second granulator. 500 g of microcrystalline cellulose, 225 g of lactose monohydrate, 225 g of crospovidone, 225 g of croscarmellose, 13 g of magnesium stearate and 27 g of talc are added and homogeneously mixed. The mixture is then homogenised for at least 20 minutes. This mixture will form part of the second, immediate-release layer of the mini-tablet. The two separate mixtures are then compressed to obtain a 4 mm double-layer mini-tablet weighing 44.3 mg. The resulting mini-tablets are then film-coated with a solution/suspension containing 440 g of ethylcellulose, 85.5 g of talc, 29 g of titanium dioxide and 15.5 g of triethyl citrate, to obtain a mini-tablet with a mean weight of 50 mg.

When subjected to disintegration and dissolution tests at pH 1, the mini-tablets remain intact for at least 2 hours, with release below 1%; at pH ≥ 6.4 they exhibit release ≤ 5% after 60 minutes; at pH 7.2 release ≤ 50% after 60 minutes; release ≤ 70% after 240 minutes, and not more than 85% after 480 minutes; the value must be > 90% after 18 hours. The following tables summarise the compositions of Examples 1-16.

### EXAMPLE 17

1.2 Kg of mesalazine is wet-granulated with an aqueous solution containing 83 g of PVP. After drying, the granulate is mixed with 105 g of hydroxypropyl methylcellulose (HPMC K4M), 54 g of hydroxypropyl methylcellulose (HPMC K100M), 2 g of polymethacrylate L100 and 2 g of polymethacrylate S100 until a homogeneous dispersion of active ingredient in the matrices is obtained; 10 g of magnesium stearate, 8 g of talc and 8 g of colloidal silicon dioxide are then added in sequence. The mixture is then homogenised for at least 15 minutes. This mixture is then compressed to obtain a tablet weighing 1472 mg. The resulting tablets are then film-coated with a gastroresistant solution/suspension based on 36 g of polymethacrylate L100, 36 g of polymethacrylate S100, 7 g of talc, 3 g of titanium dioxide and 1 g of triethyl citrate, to obtain a tablet with a mean weight of 1555 mg.

When subjected to disintegration and dissolution tests at pH 1, the tablets remain intact for at least 2 hours, with release below 1%; when subjected to the dissolution test at pH ≥ 6.4 they exhibit the following release profile: not more than 1% after 1 hour, at pH 7.2 not more than 25% after 1 hour, and not more than 40% after 2 hours; the value must be > 80% after 6 hours; and 100% after 10 hours.

### EXAMPLE 18

1.2 Kg of mesalazine is wet-granulated with an aqueous solution containing 83 g of PVP. After drying, the granulate is mixed with 106 g of hydroxypropyl methylcellulose (HPMC K4M), 53 g of hydroxypropyl methylcellulose (HPMC K100M) and 4 g of polymethacrylate L100-55 until a homogeneous dispersion of active ingredient in the matrices is obtained; 10 g of magnesium stearate, 8 g of talc and 8 g of colloidal silicon dioxide are then added in sequence. The mixture is then homogenised for at least 15 minutes. This mixture is then compressed to obtain a tablet weighing 1472 mg. The resulting tablets are film-coated with a solution/suspension of 26 g of ethylcellulose, 4 g of talc and 0.5 g of triethyl citrate. This is followed by a gastroresistant coating containing 45 g of polymethacrylate L100-55, 4 g of talc, 3 g of titanium dioxide and 0.5 g of triethyl citrate, to obtain a tablet with a mean weight of 1555 mg.

When subjected to disintegration and dissolution tests at pH 1, the tablets remain intact for at least 2 hours, with release below 1%; when subjected to the dissolution test at pH ≥ 6.4 they exhibit the following release profile: not more than 20% after 1 hour, at pH 7.2 not more than 30% after 1 hour, and not more than 50% after 2 hours; not more than 60% after 6 hours; less than 70% after 8 hours; less than 90% after 10 hours; and 100% after 18 hours.

### EXAMPLE 19

100 g of niclosamide is wet-granulated with an aqueous solution containing 15 g of PVP. After drying, the granulate is mixed with 200 g of microcrystalline cellulose, 15 g of hydroxypropyl methylcellulose (HPMC 100 lv), 35 g of hydroxypropyl methylcellulose (HPMC K100M), 2 g of polymethacrylate RL 100 and 2 g of polymethacrylate RS 100 until a homogeneous dispersion of active ingredient in the matrices is obtained; 10 g of magnesium stearate, 4 g of talc and 4 g of colloidal silicon dioxide are then added in sequence. The mixture is then homogenised for at least 15 minutes. This mixture is then compressed to obtain a tablet weighing 387 mg. This is followed by a gastroresistant coating containing 25 g of polymethacrylate L100-55, 8 g of talc, 4 g of titanium dioxide and 1 g of triethyl citrate, to obtain a tablet with a mean weight of 425 mg.

When subjected to disintegration and dissolution tests at pH 1, the tablets remain intact for at least 2 hours, with release below 1%; when subjected to the dissolution test at pH ≥ 6.4 they exhibit the following release profile: not more than 15% after 1 hour, at pH 7.2 not more than 35% after 1 hour, and not more than 50% after 2 hours; not more than 60% after 6 hours; less than 70% after 8 hours; less than 90% after 10 hours; and 100% after 18 hours.

### EXAMPLE 20

400 g of niclosamide is wet-granulated with an aqueous solution containing 35 g of PVP. After drying, the granulate is mixed with 400 g of microcrystalline cellulose, 35 g of hydroxypropyl methylcellulose (HPMC K4M), 15 g of hydroxypropyl methylcellulose (HPMC K100M), 2 g of polymethacrylate RL 100 and 2 g of polymethacrylate RS 100 until a homogeneous dispersion of active ingredient in the matrices is obtained; 10 g of magnesium stearate, 8 g of talc and 8 g of colloidal silicon dioxide are then added in sequence. The mixture is then homogenised for at least 15 minutes. This mixture is then compressed to obtain a tablet weighing 915 mg. This is followed by a gastroresistant coating containing 45 g of polymethacrylate L100-55, 5 g of talc, 4 g of titanium dioxide and 1 g of triethyl citrate, to obtain a tablet with a mean weight of 970 mg.

When subjected to disintegration and dissolution tests at pH 1, the tablets remain intact for at least 2 hours, with release below 1%; when subjected to the dissolution test at pH ≥ 6.4 they exhibit the following release profile: not more than 30% after 1 hour, at pH 7.2 not more than 40% after 1 hour, and not more than 50% after 2 hours; not more than 65% after 6 hours; less than 75% after 8 hours; less than 90% after 10 hours; and 100% after 18 hours.

### EXAMPLE 21

100 g of niclosamide is wet-granulated with an aqueous solution containing 15 g of PVP. After drying, the granulate is mixed with 100 g of microcrystalline cellulose, 100 g of calcium phosphate, 15 g of hydroxypropyl methylcellulose (HPMC 100 lv), 35 g of hydroxypropyl methylcellulose (HPMC K15M), 2 g of polymethacrylate L 100 and 2 g of polymethacrylate S 100 until a homogeneous dispersion of active ingredient in the matrices is obtained; 10 g of magnesium stearate, 4 g of talc and 4 g of colloidal silicon dioxide are then added in sequence. The mixture is then homogenised for at least 15 minutes. This mixture is then compressed to obtain a tablet weighing 387 mg. This is followed by a gastroresistant coating containing 16 g of polymethacrylate L100, 16 g of polymethacrylate S100, 6 g of talc, 3 g of titanium dioxide and 2 g of triethyl citrate, to obtain a tablet with a mean weight of 430 mg.

When subjected to disintegration and dissolution tests at pH 1, the tablets remain intact for at least 2 hours, with release below 1%; when subjected to the dissolution test at pH ≥ 6.4 they exhibit the following release profile: not more than 1% after 1 hour, at pH 7.2 not more than 30% after 1 hour, and not more than 50% after 2 hours; not more than 60% after 6 hours; less than 75% after 8 hours; less than 90% after 10 hours; and 100% after 18 hours.

### EXAMPLE 22

400 g of niclosamide is wet-granulated with an aqueous solution containing 35 g of PVP. After drying, the granulate is mixed with 150 g of microcrystalline cellulose, 150 g of calcium phosphate, 15 g of hydroxypropyl methylcellulose (HPMC 100 lv), 35 g of hydroxypropyl methylcellulose (HPMC K15M), 2 g of polymethacrylate L 100 and 2 g of polymethacrylate S 100 until a homogeneous dispersion of active ingredient in the matrices is obtained; 5 g of magnesium stearate, 8 g of talc and 8 g of colloidal silicon dioxide are then added in sequence. The mixture is then homogenised for at least 15 minutes. This mixture is then compressed to obtain a tablet weighing 810 mg. This is followed by a gastroresistant coating containing 36 g of polymethacrylate L100, 36 g of polymethacrylate S100, 8 g of talc, 3 g of titanium dioxide and 2 g of triethyl citrate, to obtain a tablet with a mean weight of 895 mg.

When subjected to disintegration and dissolution tests at pH 1, the tablets remain intact for at least 2 hours, with release below 1%; when subjected to the dissolution test at pH ≥ 6.4 they exhibit the following release profile: not more than 15% after 1 hour, at pH 7.2 not more than 30% after 1 hour, and not more than 45% after 2 hours; not more than 60% after 6 hours; less than 70% after 8 hours; less than 85% after 10 hours; and 100% after 18 hours.

**TABLE 1**

| **Active ingredient** | Triazolam F1 | Alprazolam F2 | Ramipril F3 | Ramipril F4 | Gliclazide F5 |
|---|---|---|---|---|---|
| **Ingredients of MR core** | | | | | |
| **API** | 1 | 2 | 2.5 | 2.5 | 40 |
| Lactose monohydrate | 75 | 100 | 22.5 | 22.5 | 120 |
| Microcrystalline cellulose | 30 | 40 | 74.25 | 74.25 | 22.5 |
| Dicalcium phosphate | | | | | |
| HPMC K4 M | 11 | 11 | 15 | 15 | 25 |
| HPMC K15 M | | | | | |
| HPMC K100 M | 11 | 11 | 10 | 10 | 25 |
| Eudragit L 100/55 | 0.2 | | | 2 | 0.5 |
| Eudragit L 100 | | | 0.5 | | |
| Eudragit S 100 | | | 0.5 | | |
| Shellac | | | 1 | | |
| Eudragit RL 100 | | 0.25 | | | 0.5 |
| Eudragit RS 100 | | 0.25 | | | |
| Talc | 1 | 2 | 2.5 | 2.5 | 2.5 |
| Hydrated coll. silicon | | | | | |
| dioxide | | | | | |
| PVP | | | | | |
| Mg stearate | 1 | 1.5 | 1.5 | 1.5 | 1.5 |
| Total | 130.2 | 168 | 130.25 | 130.25 | 237.5 |
| | | | | | |

| **Ingredients of IR core** | | | | | |
|---|---|---|---|---|---|
| **API** | 1 | | 2.5 | 2.5 | 40 |
| Lactose monohydrate | 25 | | 7.5 | 7.5 | 40 |
| Microcrystalline cellulose | 10 | | 24.25 | 24.25 | 7.5 |
| Crosslinked PVP | 11.6 | | 15 | 15 | 12.5 |
| Croscarmellose (AcDisol) | 11.6 | | 15 | 15 | 12.5 |
| Talc | 1 | | 2.5 | 2.5 | 2.5 |
| Mg stearate | 1 | 1.5 | 1.5 | 1.5 | 1.5 |
| Hydrated coll. silicon dioxide | | | | | |
| Total | 61.2 | 68.25 | 68.25 | 68.25 | 116.5 |
| | | | | | |

| **Film-coating ingredients** | | | | | |
|---|---|---|---|---|---|
| Talc | 8 | 6.5 | 5 | 5 | 5 |
| Eudragit L 100/55 | 32 | | | 15 | 15 |
| Eudragit L 100 | | | 7.5 | | |
| Eudragit S 100 | | | 7.5 | | |
| Shellac | | 6 | | | |
| Titanium dioxide | 2.3 | 3 | 2 | 2 | 2 |
| Triethyl citrate | 1.3 | 1.5 | 2 | 2 | 2 |
| HPMC E5 Prem. | | | | | |
| Total | 43.6 | 17 | 24 | 24 | 24 |
| | | | | | |
| Grand total | 235 | 185 | 222.5 | 222.5 | 378 |

**TABLE 2 - Mini-tablets**

| **Active ingredient** | Metformin F6 | Atenolol F7 | Atenolol F8 |
|---|---|---|---|
| **Ingredients of MR core** | | | |
| **API** | 31.25 | 5 | 5 |
| Lactose monohydrate | | | |
| Microcrystalline cellulose | 3.25 | 5 | 5 |
| Dicalcium phosphate | | 12.25 | 12.25 |
| HPMC K4 M | 2.5 | 2.25 | 2.25 |
| HPMC K15 M | 1.25 | 2.25 | 2.25 |
| HPMC K100 M | | | |
| Eudragit L 100/55 | | 0.045 | 0.045 |
| Eudragit L 100 | | | |
| Eudragit S 100 | | | |
| Shellac | | | |
| Eudragit RL 100 | 0.1 | | |
| Eudragit RS 100 | 0.1 | | |
| Talc | 0.15 | 0.225 | 0.225 |
| Hydrated coll. silicon dioxide | 0.1 | | |
| PVP | 1.5 | | |
| Mg stearate | 0.3 | 0.13 | 0.13 |
| Total | 40.5 | 27.15 | 27.15 |
| | | | |

| **Ingredients of IR core** | | | |
|---|---|---|---|
| **API** | 31.25 | 5 | 5 |
| Lactose monohydrate | | 2.25 | 2.25 |
| Microcrystalline cellulose | | 5 | 5 |
| Crosslinked PVP | 3.35 | 2.25 | 2.25 |
| Croscarmellose (AcDisol) | 3.35 | 2.25 | 2.25 |
| Talc | 0.75 | 0.27 | 0.27 |
| Mg stearate | 0.3 | 0.13 | 0.13 |
| Hydrated coll. silicon dioxide | 0.1 | | |
| Total | 40.5 | 17.15 | 17.15 |
| | | | |

| **Film-coating ingredients** | | | |
|---|---|---|---|
| Talc | 2 | 0.861 | 0.861 |
| Eudragit L100/55 | 5.4 | | 1.694 |
| Eudragit L 100 | | | |
| Eudragit S 100 | | | |
| Shellac | | | |
| Titanium dioxide | 1 | 0.29 | 0.29 |
| Triethyl citrate | 0.6 | 0.149 | 0.155 |
| HPMC E5 Prem. | | 0.4 | |
| Total | 9 | 1.3 | 3.0 |
| | | | |
| Grand total | 90 | 46 | 47.3 |

**TABLE 3 - Metformin 8 × 5 mm mini-tablets = 500 mg Atenolol 10 × 4 mm mini-tablets = 100 mg**

| **Active ingredient** | Triazolam F9 | Alprazolam F10 | Ramipril F11 | Ramipril F12 | Gliclazide F13 |
|---|---|---|---|---|---|
| **Ingredients of MR core** | | | | | |
| **API** | 1 | 2 | 2.5 | 2.5 | 40 |
| Lactose monohydrate | 75 | 100 | 22.5 | 22.15 | 120 |
| Microcrystalline cellulose | 30 | 40 | 74.25 | 74.25 | 22.5 |
| HPMC K4 M | 11 | 11 | 15 | 15 | 25 |
| HPMC K100 M | 11 | 11 | 10 | 10 | 25 |
| Eudragit L 100/5 5 | 0.2 | | | 2 | 0.5 |
| Eudragit L 100 | | | 0.5 | | |
| Eudragit S 100 | | | 0.5 | | |
| Shellac | | | 1 | | |
| Eudragit RL 100 | | 0.25 | | | 0.5 |
| Eudragit RS 100 | | 0.25 | | | |
| Talc | 1 | 2 | 2.5 | 2.5 | 2.5 |
| Mg stearate | 1 | 1.5 | 1.5 | 1.5 | 1.5 |
| Total | 130.2 | 168 | 130.25 | 130.25 | 237.5 |
| | | | | | |

| **Ingredients of IR core** | | | | | |
|---|---|---|---|---|---|
| **API** | 1 | | 2.5 | 2.5 | 40 |
| Lactose monohydrate | 25 | | 7.5 | 7.5 | 40 |
| Microcrystalline cellulose | 10 | | 24.25 | 24.25 | 7.5 |
| Crosslinked PVP | 11.6 | | 15 | 15 | 12.5 |
| Croscarmellose (AcDisol) | 11.6 | | 15 | 15 | 12.5 |
| Talc | 1 | | 2.5 | 2.5 | 2.5 |
| Mg stearate | 1 | | 1.5 | 1.5 | 1.5 |
| Total | 61.2 | | 68.25 | 68.25 | 116.5 |
| | | | | | |

| **Film-coating ingredients** | | | | | |
|---|---|---|---|---|---|
| Talc | 8 | 6.5 | 5 | 2 | 5 |
| Ethylcellulose | 12.4 | 7 | 7 | 10 | 12 |
| Titanium dioxide | 2.3 | 3 | 2 | 2 | 2 |
| Triethyl citrate | 1.3 | 1.5 | 2 | 2 | 2 |
| Total | 24 | 18 | 16 | 17 | 21 |
| | | | | | |
| Grand total | 215.4 | 186 | 214.5 | 215.5 | 375 |

**TABLE 4 - Mini-tablets**

| **Active ingredient** | Metformin F14 | Atenolol F15 | Atenolol F16 |
|---|---|---|---|
| **Ingredients of MR MiniCore** | | | |
| **API** | 31.25 | 5 | 5 |
| Microcrystalline cellulose | 3.25 | 5 | 5 |
| Dicalcium phosph. | | 12.25 | 12.25 |
| HPMC K4 M | 2.5 | 2.25 | 2.25 |
| HPMC K15 M | 1.25 | 2.25 | 2.25 |
| Eudragit L100/55 | | 0.045 | 0.045 |
| Eudragit RL 100 | 0.1 | | |
| Eudragit RS 100 | 0.1 | | |
| PVP | 1.5 | | |
| Talc | 0.15 | 0.225 | 0.225 |
| Mg stearate | 0.3 | 0.13 | 0.13 |
| Silicon dioxide | 0.1 | | |
| Total | 40.5 | 17.15 | 17.15 |
| | | | |

| **Ingredients of IR core** | | | |
|---|---|---|---|
| **API** | 31.25 | 5 | 5 |
| Microcrystalline cellulose | | 10 | 10 |
| Lactose monohydrate | | 2.25 | 2.25 |
| Crosslinked PVP | 3.35 | 2.25 | 2.25 |
| Croscarmellose (AcDisol) | 3.35 | 2.25 | 2.25 |
| Talc | 0.75 | 0.27 | 0.27 |
| Mg stearate | 0.3 | 0.13 | 0.13 |
| PVP | 1.5 | | |
| Total | 40.5 | 22.15 | 22.15 |
| | | | |

| **Film-coating ingredients** | | | |
|---|---|---|---|
| Talc | 0.2 | 0.855 | 0.855 |
| Eudragit L 100/55 | | | |
| Ethylcellulose | 5 | 2.7 | 4.4 |
| Titanium dioxide | 1 | 0.29 | 0.29 |
| Triethyl citrate | 0.6 | 0.155 | 0.155 |
| Total | 9 | 3.0 | 5.7 |
| | | | |
| Grand total | 87.8 | 48.4 | 50.0 |

| | | | |
|---|---|---|---|
| Metformin 8 × 5 mm mini-tablets = 500 mg Atenolol 10x4 mm mini-tablets = 100 mg | | | |

**Table 5**

| **Active Ingredient** | Mesalazine F17 | Mesalazine F18 | Niclosamide F19 | Niclosamide F20 | Niclosamide F21 | Niclosamide F22 |
|---|---|---|---|---|---|---|
| **Ingredients of MR core** | | | | | | |
| | 1200 | 1200 | 100 | 400 | 100 | 400 |
| Calcium phosphate | | | | | 100 | 150 |
| Microcrystalline cellulose | | | 200 | 400 | 100 | 150 |
| HPMC 100 lv | | | 15 | | 15 | 15 |
| HPMC K4 M | 105 | 106 | | 35 | | |
| HPMC K15 M | | | | | 35 | 35 |
| HPMC K100 M | 54 | 53 | 35 | 15 | | |
| Eudragit L100 / 55 | | 4 | | | | |
| Eudragit L 100 | 2 | | | | 2 | 2 |
| Eudragit S 100 | 2 | | | | 2 | 2 |
| | | | | | | |
| Eudragit RL 100 | | | 2 | 2 | | |
| Eudragit RS 100 | | | 2 | 2 | | |
| Talc | 8 | 8 | 4 | 8 | 4 | 8 |
| Hydrated coll. silicon dioxide | 8 | 8 | 4 | 8 | 4 | 8 |
| PVP | 83 | 83 | 15 | 35 | 15 | 35 |
| Mg Stearate | 10 | 10 | 10 | 10 | 10 | 5 |
| | 1472 | 1472 | 387 | 915 | 387 | 810 |
| | | | | | | |

| **Film-coating ingredients** | | | | | | |
|---|---|---|---|---|---|---|
| | | | | | | |
| Talc | 7 | 8 | 8 | 5 | 6 | 8 |
| Eudragit L100 / 55 | | 45 | 25 | 45 | | |
| Eudragit L 100 | 36 | | | | 16 | 36 |
| Eudragit S 100 | 36 | | | | 16 | 36 |
| | | | | | | |
| Ethylcellulose | | 26 | | | | |
| Titanium dioxide | 3 | 3 | 4 | 4 | 3 | 3 |
| Triethyl citrate | 1 | 1 | 1 | 1 | 2 | 2 |
| | | | | | | |
| Total | 84 | 84 | 38 | 58 | 45 | 85 |
| Grand total | 1555 | 1555 | 425 | 970 | 430 | 895 |

## Claims

1. A controlled-release solid oral pharmaceutical composition comprising one or more active ingredients in a core and an outer coating of said core, **characterised in that**:
a) the core consists of:
(i) a monolithic matrix containing an active ingredient, and a combination of at least one hydroxypropyl methylcellulose having a viscosity ranging between 3 and 5000 mPa.s 2% in H₂O at 20°C, and at least one hydroxypropyl methylcellulose having a viscosity ranging between 13500 and 280,000 mPa.s 2% in H₂O at 20°C, with at least one or two methacrylic polymers/copolymers and/or shellac, cellulose acetate phthalate, hydroxypropyl methylcellulose acetate succinate, or
(ii) a monolithic matrix as defined above adjacent to an immediate-release layer comprising the same active ingredient as contained in the monolithic matrix;
b) the coating consists of a layer comprising ethylcellulose or of a gastroresistant layer or of a layer comprising ethylcellulose which in turn is coated with gastroresistant polymers.

2. A composition as claimed in claim 1 wherein the core consists of a monolithic matrix as defined in claim 1, point (i).

3. A composition as claimed in claim 1 wherein the core consists of a monolithic matrix as defined in claim 1, adjacent to an immediate-release layer comprising the same active ingredient as contained in the monolithic matrix.

4. A composition as claimed in any one of claims 1 to 3 wherein the coating consists of a layer comprising ethylcellulose.

5. A composition as claimed in any one of claims 1 to 3 wherein the coating consists of a layer comprising ethylcellulose coated with gastroresistant polymers.

6. A composition as claimed in any one of claims 1 to 3 wherein the coating consists of a gastroresistant layer.

7. A composition as claimed in one or more of claims 1 to 6 wherein the acrylic/methacrylic polymer or copolymer is selected from pH-independent methacrylic ester copolymers, pH-independent ammonium alkyl methacrylate copolymers; amino alkyl methacrylate copolymers soluble up to pH 5.0, methacrylic acid copolymers soluble at pH ≥ 5.5, methacrylic acid copolymers soluble at pH 6.0-7.0; pH-dependent methacrylic acid copolymers soluble at pH ≥ 7.0.

8. A composition as claimed in one or more of claims 1 to 6 wherein the monolithic matrix comprises shellac.

9. A composition as claimed in one or more of claims 1 to 8 wherein the gastroresistant coating comprises pH-dependent methacrylic acid copolymers soluble at pH ≥ 5.5; pH-dependent methacrylic acid copolymers soluble at pH 6.0-7.0; pH-dependent methacrylic acid copolymers soluble at pH ≥ 7.0; shellac; cellulose acetate phthalate; cellulose succinate.

10. A composition as claimed in one or more of claims 1 to 9 wherein the hydroxypropyl methylcellulose having a viscosity ranging between 3 and 5000 mPa.s 2% in H₂O at 20°C constitutes 1 to 20% of the weight of the core, the hydroxypropyl methylcellulose having a viscosity ranging between 13500 and 280,000 mPa.s 2% in H₂O at 20°C constitutes 1 to 20% of the weight of the matrix, and the methacrylic acid polymer/copolymer constitutes 0.1 to 20% of the weight of the core.

11. A composition as claimed in one or more of claims 1-5 to 6-10 wherein ethylcellulose is present in percentages ranging from 1 to 20% of the weight of the core.

12. A composition as claimed in one or more of claims 1 to 11 wherein the active ingredient is selected from non-steroidal anti-inflammatory drugs, beta blockers, vasodilators, calcium antagonists, angiotensin II receptor antagonists, ACE inhibitors, statins, antidiabetics, hypoglycaemics, incretin mimetics, antihistamines, tranquillisers, antidepressants, antipsychotics, analgesics, antitumorals, antibacterials, antibiotics, antifungals, antihyperlipidaemics, antifibrinolytics, steroidal anti-inflammatory drugs, monoclonal antibodies, antivirals, anticoagulants, anti-rheumatics, immunosuppressants, immunomodulators, bronchodilators, multiple sclerosis drugs, antihelminthics/antiinflammatories.

## Patentansprüche

1. Feste orale pharmazeutische Zusammensetzung mit gesteuerter Wirkstofffreisetzung, die einen oder mehrere Wirkstoffe in einem Kern und einen äußeren Überzug um den Kern umfasst, **dadurch gekennzeichnet, dass**
a) der Kern aus Folgendem besteht:
(i) einer monolithischen Matrix, die einen Wirkstoff und eine Kombination von wenigstens einer Hydroxypropylmethylcellulose mit einer Viskosität im Bereich von 3 bis 5000 mPa.s mit 2% in H₂O bei 20°C und wenigstens einer Hydroxypropylmethylcellulose mit einer Viskosität im Bereich von 13500 bis 280000 mPa.s mit 2% in H₂O bei 20°C mit wenigstens einem oder zwei Methacrylsäure-Polymere/-Copolymere und/oder Schellack, Celluloseacetatphthalat, Hydroxypropylmethylcelluloseacetatsuccinat enthält, oder
(ii) einer monolithischen Matrix, wie sie oben definiert ist, angrenzend an eine Schicht mit sofortiger Wirkstofffreisetzung, die denselben Wirkstoff umfasst, wie er auch in der monolithischen Matrix enthalten ist;
b) die Beschichtung aus einer Schicht, die Ethylcellulose umfasst, oder aus einer magensaftresistenten Schicht oder aus einer Schicht, die Ethylcellulose umfasst, die wiederum von magensaftresistenten Polymeren besteht.

2. Verfahren gemäß Anspruch 1, wobei der Kern aus einer monolithischen Matrix besteht, wie sie in Anspruch 1, Punkt (i), definiert ist.

3. Zusammensetzung gemäß Anspruch 1, wobei der Kern aus einer monolithischen Matrix, wie sie in Anspruch 1 definiert ist, besteht, die an eine Schicht mit sofortiger Wirkstofffreisetzung, die denselben Wirkstoff umfasst, wie er auch in der monolithischen Matrix enthalten ist, angrenzt.

4. Zusammensetzung gemäß einem der Ansprüche 1 bis 3, wobei die Beschichtung aus einer Schicht besteht, die Ethylcellulose umfasst.

5. Zusammensetzung gemäß einem der Ansprüche 1 bis 3, wobei die Beschichtung aus einer Schicht besteht, die Ethylcellulose umfasst, welche mit magensaftresistenten Polymeren überzogen ist.

6. Zusammensetzung gemäß einem der Ansprüche 1 bis 3, wobei die Beschichtung aus einer magensaftresistenten Schicht besteht.

7. Zusammensetzung gemäß einem oder mehreren der Ansprüche 1 bis 6, wobei das Acryl-/Methacryl-Polymer oder -Copolymer aus pH-unabhängigen Methacrylsäureester-Copolymeren, pH-unabhängigen Ammoniumalkylmethacrylat-Copolymeren, Aminoalkylmethacrylat-Copolymeren, die bis zu pH 5,0 löslich sind, Methacrylsäure-Copolymeren die bei pH > 5,5 löslich sind, Methacrylsäure-Copolymeren, die bei pH 6,0-7,0 löslich sind; pH-abhängigen Methacrylsäure-Copolymeren, die bei pH > 7,0 löslich sind, ausgewählt ist.

8. Zusammensetzung gemäß einem oder mehreren der Ansprüche 1 bis 6, wobei die monolithische Matrix Schellack umfasst.

9. Zusammensetzung gemäß einem oder mehreren der Ansprüche 1 bis 8, wobei die magensaftresistente Beschichtung pH-abhängige Methacrylsäure-Copolymere, die bei pH ≥ 5,5 löslich sind, pH-abhängige Methacrylsäure-Copolymere, die bei pH 6,0-7,0 löslich sind, pH-abhängige Methacrylsäure-Copolymere, die bei pH ≥ 7,0 löslich sind, Schellack, Celluloseacetatphthalat, Cellulosesuccinat umfasst.

10. Zusammensetzung gemäß einem oder mehreren der Ansprüche 1 bis 9, wobei die Hydroxypropylmethylcellulose, die mit 2% in H₂O bei 20°C eine Viskosität im Bereich von 3 bis 5000 mPa.s aufweist, 1 bis 20% des Gewichts des Kerns ausmacht, die Hydroxypropylmethylcellulose, die mit 2% in H₂O bei 20°C eine Viskosität im Bereich von 13500 bis 280000 mPa.s aufweist, 1 bis 20% des Gewichts der Matrix ausmacht und das Methacrylsäure-Polymer/-Copolymer 0,1 bis 20% des Gewichts des Kerns ausmacht.

11. Zusammensetzung gemäß einem oder mehreren der Ansprüche 1-5 bis 6-10, wobei Ethylcellulose in Prozentanteilen im Bereich von 1 bis 20% des Gewichts des Kerns vorhanden ist.

12. Zusammensetzung gemäß einem oder mehreren der Ansprüche 1 bis 11, wobei der Wirkstoff aus nichtsteroidalen Entzündungshemmern, Betablockern, Vasodilatatoren, Calcium-Antagonisten, Angiotensin-II-Rezeptor-Antagonisten, ACE-Hemmern, Statinen, Antidiabetika, Hypoglykämika, Incretin-Mimetika, Antihistaminika, Tranquilizern, Antidepressiva, Antipsychotika, Analgetika, Antitumorwirkstoffen, antibakteriellen Wirkstoffen, Antibiotika, Antimykotika, Antihyperlipidämika, Antifibrinolytika, steroidalen Entzündungshemmern, monoklonalen Antikörpern, antiviralen Wirkstoffen, Antikoagulantien, Antirheumatika, Immunsuppressiva, Immunmodulatoren, Bronchodilatatoren, Multiple-Sklerose-Wirkstoffen, Antihelminthika/Entzündungshemmern ausgewählt ist.

## Revendications

1. Composition pharmaceutique orale solide à libération contrôlée comprenant un ou plusieurs ingrédients actifs dans un noyau et un enrobage externe dudit noyau, **caractérisée en ce que** :
a) le noyau est constitué par :
(i) une matrice monolithique contenant un ingrédient actif, et une combinaison d'au moins une hydroxypropyl méthylcellulose possédant une viscosité se situant dans une plage entre 3 et 5000 mPa.s à 2 % dans du H₂O à 20 °C et d'au moins une hydroxypropyl méthylcellulose possédant une viscosité se situant dans une plage entre 13500 et 280.000 mPa.s à 2 % dans du H₂O à 20 °C, avec au moins un ou deux polymères/copolymères méthacryliques et/ou de la gomme-laque, de l'acétophtalate de cellulose, de l'acétosuccinate d'hydroxypropyl méthylcellulose ; ou
(ii) une matrice monolithique telle que définie ci-dessus adjacente à une couche à libération immédiate comprenant le même ingrédient actif que celui contenu dans la matrice monolithique ;
b) l'enrobage est constitué d'une couche comprenant de l'éthylcellulose ou d'une couche gastrorésistante ou d'une couche comprenant de l'éthylcellulose qui à son tour est enrobée avec des polymères gastrorésistants.

2. Composition selon la revendication 1, dans laquelle le noyau est constitué d'une matrice monolithique telle que définie à la revendication 1, point (i).

3. Composition selon la revendication 1, dans laquelle le noyau est constitué d'une matrice monolithique telle que définie à la revendication 1, adjacente à une couche à libération immédiate comprenant le même ingrédient actif que celui contenu dans la matrice monolithique.

4. Composition selon l'une quelconque des revendications 1 à 3, dans laquelle l'enrobage est constitué d'une couche comprenant de l'éthylcellulose.

5. Composition selon l'une quelconque des revendications 1 à 3, dans laquelle l'enrobage est constitué d'une couche comprenant de l'éthylcellulose enrobée avec des polymères gastrorésistants.

6. Composition selon l'une quelconque des revendications 1 à 3, dans laquelle l'enrobage est constitué d'une couche gastrorésistante.

7. Composition selon une ou plusieurs des revendications 1 à 6, dans laquelle le polymère ou le copolymère acrylique/méthacrylique est choisi parmi des copolymères d'ester méthacrylique indépendants du pH, des copolymères de méthacrylate d'alkylammonium indépendants du pH, des copolymères de méthacrylate d'aminoalkyle solubles jusqu'à un pH de 5,0, des copolymères d'acide méthacrylique solubles à un pH ≥ 5,5, des copolymères d'acide méthacrylique solubles à un pH de 6,0 à 7,0, des copolymères d'acide méthacrylique dépendants du pH solubles à un pH ≥ 7,0.

8. Composition selon une ou plusieurs des revendications 1 à 6, dans laquelle la matrice monolithique comprend de la gomme-laque.

9. Composition selon une ou plusieurs des revendications 1 à 8, dans laquelle l'enrobage gastrorésistant comprend des copolymères d'acide méthacrylique dépendants du pH solubles à un pH ≥ 5,5, des copolymères d'acide méthacrylique dépendants du pH solubles à un pH de 6,0 à 7,0, des copolymères d'acide méthacrylique dépendants du pH solubles à un pH ≥ 7,0, de la gomme-laque, de l'acétophtalate de cellulose, du succinate de cellulose.

10. Composition selon une ou plusieurs des revendications 1 à 9, dans laquelle l'hydroxypropyl méthylcellulose possédant une viscosité se situant dans une plage entre 3 et 5000 mPa.s à 2 % dans du H₂O à 20 °C constitue de 1 à 20 % du poids du noyau, l'hydroxypropyl méthylcellulose possédant une viscosité se situant dans une plage entre 13500 et 280.000 mPa.s à 2 % dans du H₂O à 20 °C constitue de 1 à 20 % du poids de la matrice, et le polymère/copolymère d'acide méthacrylique constitue de 0,1 à 20 % du poids du noyau.

11. Composition selon une ou plusieurs des revendications 1 à 5 ou 6 à 10, dans laquelle l'éthylcellulose est présente dans des pourcentages se situant dans la plage de 1 à 20 % du poids du noyau.

12. Composition selon une ou plusieurs des revendications 1 à 11, dans laquelle l'ingrédient actif est choisi parmi des médicaments anti-inflammatoires non stéroïdiens, des bêtabloquants, des vasodilatateurs, des antagonistes du calcium, des antagonistes du récepteur de l'angiotensine II, des inhibiteurs d'ACE, des statines, des antidiabétiques, des hypoglycémiants, des incrétinomimétiques, des antihistaminiques, des tranquillisants, des antidépresseurs, des antipsychotiques, des analgésiques, des antitumoraux, des antibactériens, des antibiotiques, des antifongiques, des antihyperlipidémiants, des antifibrinolytiques, des médicaments anti-inflammatoires stéroïdiens, des anticorps monoclonaux, des antiviraux, des anticoagulants, des antirhumatismaux, des immunosuppresseurs, des immunomodulateurs, des bronchodilatateurs, des médicaments contre la sclérose en plaques, des antihelminthiques/anti-inflammatoires.
